# EUROPÄISCHE PATENTSCHRIFT

(11) **EP 3 183 031 B1**
(45) Veröffentlichungstag und Bekanntmachung des Hinweises auf die Patenterteilung: **02.01.2019**
(21) Anmeldenummer: 15788022.0
(22) Anmeldetag: 30.10.2015
(51) Int. Cl.: A61N 1/36, A61M 21/00, A61N 2/00, A61B 5/00

(54) **VORRICHTUNG ZUR EFFEKTIVEN NICHT-INVASIVEN DESYNCHRONISIERENDEN NEUROSTIMULATION**
DEVICE FOR EFFECTIVE NON-INVASIVE DESYNCHRONIZING NEUROSTIMULATION
DISPOSITIF DE NEUROSTIMULATION DÉSYNCHRONISANTE EFFICACE NON INVASIVE

(30) Priorität: 03.11.2014 DE 102014115997
(43) Veröffentlichungstag der Anmeldung: 28.06.2017
(73) Patentinhaber: Forschungszentrum Jülich GmbH, 52425 Jülich (DE)
(72) Erfinder: TASS, Peter Alexander, 83684 Tegernsee (DE); POPOVYCH, Oleksandr, 52355 Düren (DE); XENAKIS, Markos, 15121 Athens (GR)
(74) Vertreter: Müller-Boré & Partner Patentanwälte PartG mbB
(86) Internationale Anmeldenummer: PCT/EP2015/075297
(87) Internationale Veröffentlichungsnummer: WO 2016/071236

(56) Entgegenhaltungen:
- DE-A1-102004 025 945
- DE-A1-102008 012 669
- DE-A1-102008 052 078
- DE-A1-102009 025 407
- DE-A1-102010 016 404
- HAUPTMANN C ET AL: "External trial deep brain stimulation device for the application of desynchronizing stimulation techniques; External trial deep brain stimulation device", JOURNAL OF NEURAL ENGINEERING, INSTITUTE OF PHYSICS PUBLISHING, BRISTOL, GB, Bd. 6, Nr. 6, Dezember 2009 (2009-12), Seite 66003, XP020170009, ISSN: 1741-2552

## Beschreibung

Die Erfindung betrifft eine Vorrichtung zur effektiven nicht-invasiven desynchronisierenden Neurostimulation.
Bei Patienten mit neurologischen oder psychiatrischen Erkrankungen, z. B. Morbus Parkinson, essentiellem Tremor, Dystonie, Funktionsstörungen nach Schlaganfall, Migräne, Zwangserkrankungen, Epilepsie, Tinnitus, Schizophrenie, Depression, Borderline Persönlichkeitsstörung sowie Reizdarmsyndrom, sind Nervenzellverbände in umschriebenen Bereichen des Gehirns krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.
Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalgaglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Beim chronisch subjektiven Tinnitus findet sich krankhafte synchrone Aktivität in einem Netzwerk von auditorischen sowie nicht-auditorischen Hirnarealen.

Bei Patienten mit Hirn- und/oder Rückenmarkserkrankungen, welche durch übermäßig synchronisierte neuronale Aktivität gekennzeichnet sind, werden nicht-invasiv bestimmte raum-zeitliche Reizmuster, insbesondere die "Coordinated Reset"-Stimulation (CR-Stimulation) appliziert, um eine dauerhafte Linderung zu erzielen. Die nicht-invasive CR-Stimulation kann mittels verschiedener Stimulationsmodi realisiert werden:
(i) durch sensorische Reizung, d. h. durch physiologische Reizung von Rezeptoren, wie z. B. akustische Reizung des Innenohrs, visuelle Reizung der Retina oder mechanische (z. B. vibrotaktile) oder thermische Reizung von Haut-, Unterhaut-, Muskel- und Sehnenrezeptoren;
(ii) durch Reizung peripherer Nerven (und zugehöriger Rezeptoren) z. B. mittels elektrischem Strom (z. B. transkutane Elektrostimulation), mittels Magnetfeldern (transdermale Magnetstimulation) oder mittels Ultraschall; und
(iii) durch Reizung des Gehirns oder Rückenmarks z. B. mittels elektrischem Strom (z. B. externe kraniale bzw. transkranielle Neurostimulation), mittels Magnetfeldern (z. B. transkranielle Magnetstimulation) oder mittels Ultraschall.

Zur Behandlung des chronisch subjektiven tonalen bzw. engbandigen Tinnitus wird die akustische CR-Stimulation verwandt. Hierzu werden Therapietöne an den dominanten Tinnituston angepasst und im Sinne der CR-Stimulation appliziert, um eine lang anhaltende, das Ausschalten der Stimulation deutlich überdauernde Desynchronisation der krankhaft synchronen Aktivität bzw. sogar eine anhaltende Desynchronisation derselben zu erzielen. Die akustische CR-Stimulation zur Behandlung des Tinnitus bewirkt eine signifikante und deutlich ausgeprägte Abnahme der Symptomatik (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137-159 (2012)), eine signifikante Abnahme der krankhaften neuronalen Synchronisation in einem Netzwerk von auditorischen und nicht-auditorischen Hirnarealen (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137-159 (2012); I. Adamchic, T. Toth, C. Hauptmann, P. A. Tass: Reversing pathologically increased EEG power by acoustic CR neuromodulation. Human Brain Mapping 35 (2014) 2099-2118), eine signifikante Abnahme der krankhaften Interaktionen zwischen unterschiedlichen Hirnarealen im selben (vgl. A. N. Silchenko, I. Adamchic, C. Hauptmann, P. A. Tass: Impact of acoustic coordinated reset neuromodulation on effective connectivity in a neural network of phantom sound. Neuroimage 77, 133-147 (2013)) sowie in unterschiedlichen (vgl. I. Adamchic, B. Langguth, C. Hauptmann, P. A. Tass: Abnormal brain activity and cross-frequency coupling in the tinnitus network. Frontiers in Neuroscience 8, 284 (2014)) Frequenzbereichen.

In analoger Weise lässt sich die Parkinsonsche Erkrankung mittels vibrotaktiler CR-Stimulation behandeln. Weitere Indikationen stellen z. B. Epilepsien, Funktionsstörungen nach Schlaganfall, chronische Schmerzsyndrome (mittels vibrotaktiler und/oder thermischer CR-Stimulation), Migräne (z. B. mittels visueller CR-Stimulation) dar. Des Weiteren lassen sich diese Erkrankungen mit transkranieller Magnetstimulation oder direkter elektrischer Stimulation des Gehirns oder direkter Hirnstimulation mittels Ultraschall behandeln.

Für die oben aufgeführten Stimulationsmodalitäten (i) bis (iii) sollte aus den im Folgenden aufgeführten Gründen zur Vermeidung von Nebenwirkungen und/oder zur Steigerung der therapeutischen Wirksamkeit mit Reizen von geringer Intensität stimuliert werden können:
(i) Bei der sensorischen Stimulation ist es wichtig, die gewünschten Stimulationseffekte (z. B. eine Phasenrücksetzung der krankhaft synchronisierten oszillatorischen Aktivität im Gehirn bzw. Rückenmark) bei möglichst geringer Reizstärke überhaupt erzielen zu können. Z. B. müssen bei der akustischen CR-Stimulation zur Behandlung des Tinnitus typischerweise schwerhörige Patienten behandelt werden. Die Stimulation mit lauten Tönen kann das Innenohr schädigen, die Kommunikation mit anderen erschweren sowie Warnlaute (z. B. Fahrzeughupe, Fahrradklingel) verdecken bzw. vom Patienten infolge der vergleichsweise nahe an der Hörschwelle verlaufenden Unerträglichkeitsschwelle als deutlich unangenehm empfunden werden, und die laute Stimulation kann auch von der Umgebung des Patienten gehört und als störend empfunden werden. Bei der visuellen CR-Stimulation kann es insbesondere bei Migränepatienten zu unangenehmen Blendeffekten kommen. Bei der mechanischen, z. B. vibrotaktilen oder thermischen CR-Stimulation von Patienten mit chronischen Schmerzsyndromen (z. B. mit Morbus Sudeck oder Neuralgien) können schon leichtere Berührungen oder Wärmereize als unangenehm oder sogar schmerzhaft empfunden werden. Wenn in derartigen Fällen z. B. über die kontralaterale Extremität oder Gesichts- bzw. Körperhälfte behandelt werden muss, ist die Reizwirkung infolge der Applikation in der gesunden Körperhälfte nicht stark ausgeprägt. Insgesamt ist es bei der sensorischen CR-Stimulation sehr vorteilhaft, wenn mit sehr geringen Reizstärken stimuliert werden kann, da sensorische Reize (z. B. Töne, Helligkeitsschwankungen von Transmissionsbrillen etc.) die physiologische Reizverarbeitung stören können.
(ii) Um bei der elektrischen oder magnetischen Reizung peripherer Nerven möglichst fokal stimulieren zu können und Nebenwirkungen, die durch die Mitreizung benachbarter Strukturen hervorgerufen werden (z. B. Muskelkontraktionen, Schmerzempfindungen etc.), vermeiden zu können, ist es wichtig, möglichst geringe Reizstärken zu verwenden.
(iii) Sowohl die elektrische als auch die magnetische Reizung des Gehirns oder Rückenmarks sind nicht sehr fokal. Insbesondere führt die direkte elektrische Stimulation des Gehirns selbst im günstigsten Falle der Stimulation über eine Vielzahl von kleinen Elektroden und bei Verwendung aufwändiger Kopfmodelle neben einer fokalen starken Reizung zu einer Mitreizung von weit ausgedehnten Hirngebieten, die gerade bei chronischer Reizung unbedingt vermieden bzw. verringert werden sollte. In gleicher Weise sollte die Ultraschallreizung auf die tatsächlichen Zielgebiete im Gehirn beschränkt werden.

In all diesen Fällen ist es also nötig, bei möglichst geringen Reizstärken behandeln zu können, um die unerwünschte Mitreizung von Nicht-Zielarealen zu verringern. Dies allerdings führt häufig dazu, dass die Behandlung nicht hinreichend wirksam ist.

Die Publikation "External trial deep brain stimulation device for the application of desynchronizing stimulation techniques; External trial deep brain stimulation device" von Hauptmann et al., Journal of Neural Engineering, Bd. 6, Nr. 6, Dezember 2009, Seite 66003, offenbart eine Vorrichtung zur Unterdrückung einer krankhaft synchronen und oszillatorischen neuronalen Aktivität, die eine nicht-invasive Stimulationseinheit, eine Messeinheit und eine Steuer- und Analyseeinheit umfasst, wobei die Stimulationsparameter zur Verbesserung des Stimulationserfolgs verändert werden.
DE 10 2009 025407 A1 offenbart eine Vorrichtung mit einer Steuereinheit und einer Mehrzahl von Stimulationseinheiten, die in den Körper eines Patienten implantiert sind und optische Reize erzeugen, wobei die optischen Reize bei der Stimulation von Neuronen, die eine krankhaft synchrone und oszillatorische neuronale Aktivität aufweisen, die Phase der neuronalen Aktivität der Neuronen zurücksetzen und die Steuereinheit die Stimulationseinheiten derart ansteuert, dass mindestens zwei der Stimulationseinheiten die Phasen der jeweils stimulierten Neuronen zu unterschiedlichen Zeitpunkten zurücksetzen.
DE 10 2008 052078 A1 offenbart eine Vorrichtung mit einer ersten Stimulationseinheit zur Erzeugung von elektrischen ersten Reizen, die bei einer Verabreichung an das Gehirn und/oder Rückenmark eines Patienten eine krankhaft synchrone Aktivität von Neuronen im Gehirn und/oder Rückenmark des Patienten unterdrücken, einer zweiten Stimulationseinheit zur Erzeugung von optischen und/oder akustischen und/oder taktilen und/oder vibratorischen zweiten Reizen, und einer Steuereinheit zur Steuerung der ersten und zweiten Stimulationseinheit, wobei die Erzeugung der ersten und zweiten Reize wahlweise in einem ersten oder einem zweiten Betriebsmodus erfolgt, und die Steuereinheit die erste und zweite Stimulationseinheit derart ansteuert, dass im ersten Betriebsmodus die Erzeugung von mindestens 60% der zweiten Reize zeitlich an die Erzeugung der ersten Reize gekoppelt ist und im zweiten Betriebsmodus die Erzeugung von mindestens 60% der zweiten Reize ohne die Erzeugung der ersten Reize erfolgt.

Der Erfindung liegt daher die Aufgabe zugrunde, eine Vorrichtung anzugeben, die es ermöglichen, durch Reizung mit minimalsten Reizstärken gute und insbesondere lang anhaltende therapeutische Effekte zu erzielen.

Die der Erfindung zugrunde liegende Aufgabenstellung wird durch die Merkmale des unabhängigen Anspruchs 1 gelöst. Vorteilhafte Weiterbildungen und Ausgestaltungen der Erfindung sind in den Unteransprüchen angegeben.
Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur Unterdrückung einer krankhaft synchronen und oszilla-torischen neuronalen Aktivität und insbesondere zur De-synchronisierung von Neuronen mit einer krankhaft syn-chronen und oszillatorischen Aktivität gemäß einer ers-ten Ausgestaltung;
- Fig. 2: eine schematische Darstellung einer Vorrichtung zur Unterdrückung einer krankhaft synchronen und oszilla-torischen neuronalen Aktivität und insbesondere zur De-synchronisierung von Neuronen mit einer krankhaft syn-chronen und oszillatorischen Aktivität gemäß einer zwei-ten Ausgestaltung;
- Fig. 3: ein Flussdiagramm zur Veranschaulichung einer Rege-lung der Längen von Stimulationsphasen und Stimulati-onspausen gemäß einer ersten Variante;
- Fig. 4: ein Flussdiagramm zur Veranschaulichung einer Rege-lung der Längen von Stimulationsphasen und Stimulati-onspausen gemäß einer zweiten Variante;
- Fig. 5: eine schematische Darstellung einer Vorrichtung zur akustischen Stimulation von Neuronen mit einer krank-haft synchronen und oszillatorischen neuronalen Aktivi-tät;
- Fig. 6: eine schematische Darstellung einer Vorrichtung zur visuellen Stimulation von Neuronen mit einer krankhaft synchronen und oszillatorischen neuronalen Aktivität;
- Fig. 7: eine schematische Darstellung einer Vorrichtung zur taktilen, vibratorischen, thermischen, elektrischen trans-kutanen und/oder magnetischen Stimulation und/oder Ultraschall-Stimulation von Neuronen mit einer krank-haft synchronen und oszillatorischen neuronalen Aktivi-tät;
- Fig. 8: eine schematische Darstellung einer CR-Reizfolge zur Stimulation einer Neuronenpopulation;
- Fig. 9: Graphen zur Veranschaulichung einer effektiven und einer ineffektiven CR-Stimulation;
- Fig. 10 bis 14: Graphen zur Veranschaulichung von CR-Stimulationen mit unterschiedlichen Stimulationsphasenlängen und Stimulationspausenlängen; und
- Fig. 15: ein Diagramm zur Darstellung der Effektivität von CR-Stimulationen mit unterschiedlichen Stimulationspha-senlängen und Stimulationspausenlängen.

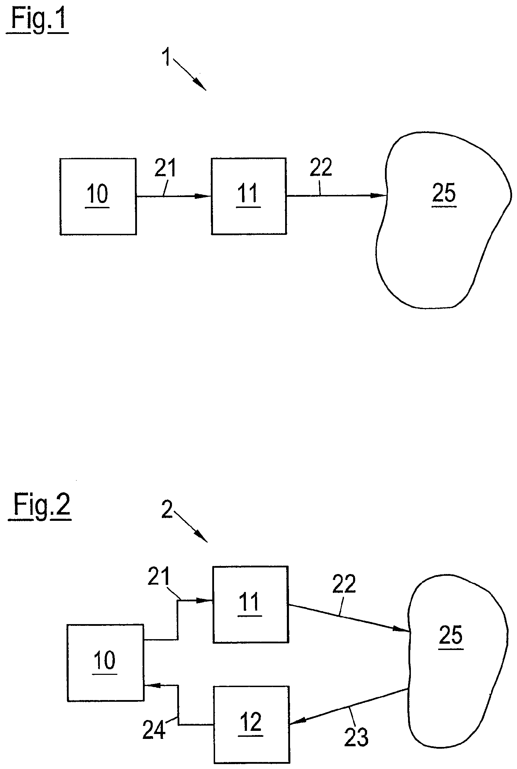

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuer- und Analyseeinheit 10 und einer Stimulationseinheit 11. Während des Betriebs der Vorrichtung 1 führt die Steuer- und Analyseeinheit 10 eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11 erzeugt anhand der Steuersignale 21 Reize 22, die einem Patienten verabreicht werden. Die Reize 22 können sensorische Reize, z. B. akustische, visuelle, taktile, vibratorische, thermische, olfaktorische, gustatorische, magnetische und/oder elektrische transkranielle, magnetische und/oder elektrische transkutane Reize und/oder Ultraschall-Reize, sein. Die Reize 22 können vom Patienten insbesondere bewusst wahrnehmbar sein. Die Reize 22 sind dazu ausgelegt, bei einer Verabreichung an den Patienten die krankhaft synchrone und oszillatorische neuronale Aktivität zu unterdrücken und insbesondere die Neuronen mit der krankhaft synchronen und oszillatorischen Aktivität zu desynchronisieren. Die thermischen Reize 22 können insbesondere durch Laserlicht erzeugt werden.

Die Stimulationseinheit 11 und insbesondere auch die Steuer- und Analyseeinheit 10 sind nicht-invasive Einheiten, d. h., während des Betriebs der Vorrichtung 1 befinden sie sich außerhalb des Körpers des Patienten und werden nicht operativ in den Körper des Patienten implantiert.

Während des Betriebs der Vorrichtung 1 kann, falls ein ungenügender Stimulationseffekt festgestellt wird, die Effizienz der Stimulation durch die Einfügung von Stimulationspausen, beispielsweise durch den Arzt oder Anwender, bei geringen Reizstärken verbessert werden. Während der Stimulationspausen findet keine Applikation von Reizen statt, welche die krankhaft synchrone und oszillatorische neuronale Aktivität unterdrücken könnten. Es ist jedoch denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Gemäß einer weiteren Ausführungsform wird während der Stimulationspausen auf Stimulation jeglicher Art mit Hilfe der Stimulationseinheit 11 verzichtet. Weiterhin können z. B. im Fall von Nebenwirkungen die vorstehend beschriebenen Stimulationspausen eingefügt werden, um eine effiziente Stimulation bei geringen Reizstärken zu ermöglichen. Die Dauer der Stimulationspausen kann konstant gehalten, durch den Arzt oder Anwender eingestellt oder wie weiter unten beschrieben geregelt werden.

Die Erfindung nutzt einen kontraintuitiven Zusammenhang: Je geringer der durch die schwache Stimulation erzielte Erfolg ist, desto längere Pausen werden in den Stimulationsvorgang eingeschoben. Insbesondere kann durch das Einschieben von derartigen Pausen bewirkt werden, dass eine ansonsten wirkungslose Stimulation effektiv ist.

Der der Erfindung zugrunde liegende kontraintuitive Mechanismus lässt sich durch die folgenden Überlegungen plausibel machen. Infolge der synaptischen Plastizität sind Verbände von Neuronen sehr plastisch, d. h., sie können in einer Vielzahl von unterschiedlichen stabilen Zuständen vorliegen. Z. B. in Zuständen mit niedriger mittlerer synaptischer Verbindungsstärke und unsynchroner neuronaler Aktivität, d. h., die Neuronen feuern auf unkorrelierte Weise, oder in Zuständen mit stark ausgeprägter mittlerer synaptischer Verbindungsstärke und synchroner neuronaler Aktivität, d. h., die Neuronen feuern auf korrelierte Weise, z. B. im Takt, also koinzident. Zwischen diesen beiden Extremen gibt es typischerweise eine Vielzahl stabiler Zustände mit intermediärer mittlerer synaptischer Verbindungsstärke und intermediärer Ausprägung der neuronalen Synchronisation. Es liegt also im mathematischen Sinn eine Multistabilität vor. Die Erfindung nutzt die überraschende Tatsache aus, dass das System, d. h. die stimulierte Neuronenpopulation, selbst mit schwacher Stimulation von einem Attraktor (stabilen Zustand) zum nächsten geschoben werden kann, wenn zwischen den Stimulationsphasen eine hinreichend lange Pause ist, während der das System in den neuen Attraktor spontan (d. h. ohne Stimulation) hineingezogen wird, was unter Stimulation nicht möglich wäre. Durch die portionierte Stimulation gelangt das System quasi schrittweise von stark synchronen Attraktoren zu zunehmend schwächer synchronen Attraktoren.

Das Einfügen hinreichend langer Stimulationspausen ermöglicht eine effiziente Stimulation bei geringen Reizstärken. Die Reizstärke kann dann bis zu einem Faktor 2 bis 3 mal geringer sein als die Mindestreizstärke, welche bei dauerhafter Stimulation, d. h. bei einer Stimulation ohne die hierin beschriebenen Stimulationspausen, zu einer lang anhaltenden Desynchronisation führt. Insbesondere kann die Reizstärke der erfindungsgemäßen Stimulation mit Stimulationspausen in einem Bereich von 1/3 der Mindestreizstärke bis zu 2/3 der Mindestreizstärke, welche bei einer dauerhaften Stimulation ohne die erfindungsgemäßen Stimulationspausen zu einer lang anhaltenden Desynchronisation führt, liegen.

Die Länge einer Stimulationspause zwischen zwei aufeinander folgenden Stimulationsabschnitten kann mindestens 3 Minuten betragen, kann aber auch wesentlich länger sein und beispielsweise mindestens 5 Minuten oder mindestens 10 Minuten oder mindestens 20 Minuten oder mindestens 30 Minuten oder mindestens 1 Stunde oder mindestens 2 Stunden oder mindestens 3 Stunden betragen. Um erste Effekte zu erzielen, muss die Stimulationspausenlänge mindestens ca. 200 Perioden der zu desynchronisierenden Oszillation entsprechen. Eine ausgeprägte Desynchronisation lässt sich erst ab ca. 1.000 bis sogar 22.000 Perioden erzielen. Im Falle einer Delta-Oszillation mit einer Frequenz im Bereich von 1 bis 4 Hz beträgt die Periodenlänge z. B. 500 ms bei 2 Hz. D. h., gute Effekte ergeben sich bei Pausen im Minuten- oder sogar Stundenbereich (1.000 bzw. 22.000 Perioden entsprechen dann ca. 8.3 min bzw. 3 Stunden). Die Peri-ode der pathologischen Oszillation kann beispielsweise am Patienten gemessen werden, es können aber auch Literatur- oder Erfahrungswerte verwendet werden.

Weiterhin kann vorzugsweise neben der Länge der Stimulationspausen auch die Länge der Stimulationsphasen, in denen eine Stimulation stattfindet, eingestellt werden, um bei geringen Reizstärken die Effizienz der Stimulation zu verbessern. Die Länge der Stimulationsphasen kann in gleicher Weise wie die Länge der Stimulationspausen konstant gehalten, durch den Arzt oder Anwender eingestellt oder wie weiter unten beschrieben geregelt werden.

Vorzugsweise können die Stimulationspausen bei zu geringem Stimulationseffekt verlängert und die Stimulationsphasen ebenfalls verlängert werden.

Beispielsweise können die Stimulationspausen und Stimulationsphasen jeweils gleich lang sein und somit gleichermaßen anwachsen. Weiterhin können die Stimulationsphasen auch zu Beginn kürzer als die Stimulationspausen sein und bei zu geringem Stimulationseffekt überproportional wachsen. Ferner kann jede geeignete andere Relation zwischen der Dauer der Stimulationspausen und der Dauer der Stimulationsphasen eingestellt werden.

In Fig. 2 ist schematisch eine Vorrichtung 2 zur Stimulation von Neuronen mit einer krankhaft synchronen und oszillatorischen neuronalen Aktivität dargestellt. Die Vorrichtung 2 stellt eine Weiterbildung der in Fig. 1 dargestellten Vorrichtung 1 dar. Die Vorrichtung 2 weist genauso wie die Vorrichtung 1 eine Steuer- und Analyseeinheit 10 und eine nicht-invasive Stimulationseinheit 11 auf. Während des Betriebs der Vorrichtung 1 führt die Steuer- und Analyseeinheit 10 eine Steuerung der Stimulationseinheit 11 durch. Dazu erzeugt die Steuer- und Analyseeinheit 10 Steuersignale 21, die von der Stimulationseinheit 11 entgegengenommen werden.

Wie oben beschrieben erzeugt die Stimulationseinheit 11 anhand der Steuersignale 21 Reize 22, die einem Patienten verabreicht werden. Die Reize 22 können sensorische Reize sein, z. B. akustische, visuelle, taktile, vibratorische, thermische, olfaktorische, gustatorische, magnetische und/oder elektrische transkutane Reize und/oder Ultraschall-Reize.

Darüber hinaus umfasst die Vorrichtung 2 eine Messeinheit 12. Der durch die Reize 22 erzielte Stimulationseffekt wird mit Hilfe der Messein-heit 12 überwacht. Die Messeinheit 12 nimmt ein oder mehrere am Patienten gemessene Messsignale 23 auf, wandelt diese gegebenenfalls in elektrische Signale 24 um und führt sie der Steuer- und Analyseeinheit 10 zu. Insbesondere kann mittels der Messeinheit 12 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 12 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 12 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. Elektroenzephalographie (EEG)-Elektroden, Magnetenzephalographie (MEG)-Sensoren und Sensoren zur Messung lokaler Feldpotentiale (LFP). Die neuronale Aktivität kann auch indirekt durch Messung der damit einhergehenden Muskelaktivität mittels Elektromyographie (EMG) oder indirekt durch Messung der Aktivierung des autonomen Nervensystems mittels Messung des Hautleitwiderstands ermittelt werden.

Alternativ können die Sensoren in den Körper des Patienten implantiert sein. Als invasive Sensoren können beispielsweise epikortikale Elektroden, Tiefenhirnelektroden zur Messung von z. B. lokalen Feldpotentialen, sub-oder epidurale Hirnelektroden, subkutane EEG-Elektroden und sub- oder epidurale Rückenmarkselektroden dienen.

Die Steuer- und Analyseeinheit 10 verarbeitet die Signale 24, z. B. können die Signale 24 verstärkt und/oder gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 z. B. einen Prozessor (z. B. einen Mikrocontroller) enthalten.

Die Steuer- und Analyseeinheit 10 überprüft anhand der in Reaktion auf die Applikation der Reize aufgenommenen Messsignale den Stimulationserfolg und stellt die Stimulationsparameter, insbesondere die Längen der oben im Zusammenhang mit Fig. 1 beschriebenen Stimulationspausen, in Abhängigkeit vom Stimulationserfolg ein. Im Fall von Nebenwirkungen und/oder generell bei ungenügendem Stimulationseffekt kann im Betrieb bei geringen Reizstärken die Effizienz der Stimulation durch die Anpassung der Stimulationspausen verbessert werden. Die Dauer der Stimulationspausen und die Dauer der Stimulationsphasen kann bei zu geringem Stimulationseffekt so geregelt werden, dass sich wieder ein Stimulationseffekt einstellt.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 12, z. B. EEG-Elektroden, gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Reize 22 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sogenannten minimalen klinisch wahrnehmbaren Verbesserung) sind dem Fachmann bekannt.

Es können in abwechselnder Reihenfolge Stimulationsphasen, in denen das Hirn und/oder Rückenmark 25 des Patienten mit den von der Stimulationseinheit 11 erzeugten Reizen 22 stimuliert wird, und Stimulationspausen, in denen keine Reize 22 appliziert werden, eingehalten werden.

Für die Regelung der Dauer L_{Stim} der Stimulationsphasen und der Dauer L_{Pause} der Stimulationspausen können z. B. Standardverfahren der Zweigrößenregelung verwandt werden. Es kann aber auch medizinisches a priori-Wissen verwandt werden, wobei die Längen L_{Stim} und L_{Pause} von einem Startwert in der (L_{Stim}, L_{Pause})-Ebene aus mit konstanter oder sukzessiv anwachsender oder deterministisch und/oder chaotisch variierter Schrittweite entlang einer Geraden oder gebogenen Kurve vergrößert werden. Beispielsweise kann das Verhältnis L_{Stim}/L_{Pause} im Rahmen dieses Regelungsvorgangs von 1/n auf n anwachsen, wobei n beispielsweise eine Zahl im Bereich von 2 bis 10 ist, z. B. 3 oder 4 oder 5.

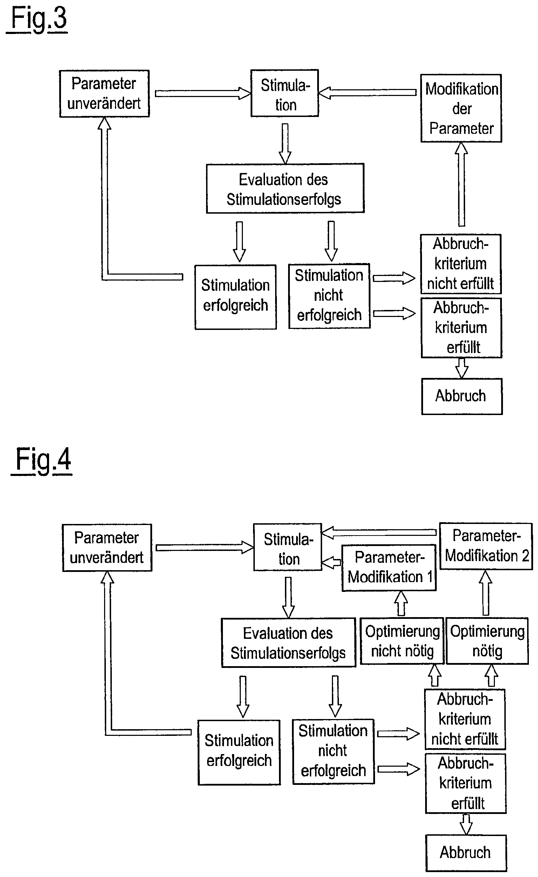

Fig. 3 zeigt ein Flussdiagramm für eine beispielhafte Regelung der Längen L_{Stim} und L_{Pause} der Stimulationsphasen und -pausen gemäß einer ersten Variante. Die Längen L_{Stim} und L_{Pause} können beispielsweise während des gesamten Vorgangs gleich groß sein, d. h., es gilt Lstim = L_{Pause} = A. Als Stellgröße für das Regelungsverfahren kann aber auch das Verhältnis L_{Stim}/L_{Pause} herangezogen werden. In letzterem Fall kann die Länge L_{Stim} beispielsweise um bis zu ±5 % oder bis zu ±10 % oder bis zu ±25 % von der Länge L_{Pause} abweichen, d. h., es gilt L_{Stim} = (1+ε)L_{Pause} = A, wobei ε in den vorstehenden genannten Fällen bis zu ±0,05 bzw. ±0,1 bzw. ±0,25 beträgt.

Der Parameter A wird von einem voreingestellten Startwert aus so lange konstant gehalten, bis die Steuer- und Analyseeinheit 10 die Stimulation als nicht erfolgreich klassifiziert. Dann wird der Parameter A insbesondere schrittweise vergrößert, bis die Steuer- und Analyseeinheit 10 anhand der von der Messeinheit 12 aufgenommenen Messsignale 24 feststellt, dass die Stimulation wieder hinreichend erfolgreich ist.

Gemäß einer Ausgestaltung wird der Parameter A bei nicht ausreichendem Stimulationserfolg so lange insbesondere schrittweise vergrößert, bis ein hinreichender Stimulationserfolg festgestellt wird oder ein Abbruchkriterium erfüllt ist. Das Abbruchkriterium soll ermitteln, wann trotz hinreichend großem und hinreichend vertretbarem Aufwand kein ausreichender Stimulationserfolg zu erwarten ist.

Das Abbruchkriterium kann z. B. erfüllt sein, wenn mindestens eines von mehreren Kriterien erfüllt ist. Ein Abbruchkriterium K1 kann z. B. erfüllt sein, wenn eine vorgegebene Behandlungsdauer, z. B. von 12 Wochen, überschritten wird, und ist ansonsten nicht erfüllt. Die Wahl von der vorgegebenen Behandlungsdauer hängt vom jeweiligen Krankheitsbild bzw. - stadium ab und spiegelt die klinische Erfahrung wider.

Fig. 4 zeigt ein Flussdiagramm für eine weitere beispielhafte Regelung der Längen Lstim und L_{Pause} der Stimulationsphasen und -pausen gemäß einer zweiten Variante. Die in Fig. 4 dargestellte Regelung ist in vielen Teilen identisch mit der Regelung aus Fig. 3, ist aber in folgender Hinsicht komplexer. Empirisch hat sich gezeigt, dass die in Fig. 3 gezeigte Regelung robust funktioniert. Es kann aber typischerweise deutlich an Zeit gespart werden, wenn - wie oben beschrieben - das Verhältnis L_{Stim}/L_{Pause} im Rahmen dieses Regelungsvorgangs von 1/n auf n angepasst an den Stimulationserfolg anwächst, wobei n beispielsweise eine Zahl im Bereich von 2 und 10 ist, z. B. 3 oder 4 oder 5. Sollte diese Anpassungsregel nicht hinreichend funktionieren, ist das Kriterium "Optimierung nötig" erfüllt. In diesem Falle wechselt die erfindungsgemäße Vorrichtung zu dem in Fig. 3 dargestelltem noch robusteren Regel-Verfahren. Konkret ist das Kriterium "Optimierung nötig" analog zu einem Abbruchkriterium, d. h., es stellt sich in einer bestimmten Zeit bzw. nach einer bestimmten Anzahl von Regelschritten kein hinreichend stark ausgeprägter therapeutischer Erfolg ein.

Gemäß einer Ausgestaltung ist ein Optimierungskriterium K2 erfüllt, wenn mittels invasiver und/oder nicht-invasiver Sensoren gemessene Biomarker und/oder Selbstbeurteilungsskalen, z. B. Befindlichkeitsskalen oder Lebensqualitätsskalen, die z .B. über ein mobiles Gerät (wie ein iPhone) eingegeben und entsprechend evaluiert werden, sich nicht hinreichend verbessern. Für die hier verwendeten invasiven und/oder nicht invasiven Sensoren können die Sensoren der Messeinheit 12 verwendet werden. Insbesondere können unterschiedliche Formen von Elektroden, z. B. Tiefenelektroden oder epikortikale Elektroden, als invasive Sensoren eingesetzt werden. Als nicht-invasive Sensoren kommen z. B. chronisch oder intermittent genutzte EEG-Elektroden oder Akzelerometer zur Detektion charakteristischer Bewegungsmuster, wie z. B. Tremor, Akinese oder epileptische Anfälle, infrage. Ein Biomarker ist z. B. die spektrale Dichte in einem charakteristischen, dem Fachmann bekannten Frequenzbereich (z. B. dem von ca. 8 bis 30 Hz verlaufenden Beta-Band beim Parkinsonpatienten) des über Tiefenelektroden abgeleiteten lokalen Feldpotentials.

Wenn der oder die Biomarker bzw. die Selbstbeurteilungsskalen (i) nicht innerhalb einer vorgegebenen Zeit, z. B. 4 Wochen, um einen bestimmten Prozentsatz vom Ausgangswert, z. B. 20 % oder 50 % im mehrtägigen Mittel je nach Erkrankung bzw. Krankheitsstadium, abnehmen und/oder (ii) nicht nach dem m-ten Anpassungsschritt des Parameters A, z. B. m = 3, um diesen Prozentsatz vom Ausgangswert abgenommen haben, ist gemäß einer Ausgestaltung das Kriterium K2 erfüllt. Ansonsten ist das Kriterium K2 nicht erfüllt. Hierbei sind die Anpassungsschritte von A konstant oder sukzessiv anwachsend oder werden deterministisch und/oder chaotisch variiert. Die Wiederholungszahl m entspricht der klinischen Erfahrung, d. h. der Zeitskala, auf der der therapeutische Erfolg sich bei der jeweiligen Erkrankung einstellen kann.

Anstelle der in den Fig. 3 und 4 dargestellten Regelungen kann auch eine Stimulation durchgeführt werden, bei der die Längen Lstim der Stimulationsphasen und die Längen L_{Pause} der Stimulationspausen konstant sind, d. h., L_{Stim} = L_{Pause} = A oder L_{Stim} = (1+ε)L_{Pause} = A mit ε im Bereich von ±0,05, ±0,1 oder ±0,25, und die Stimulation mit konstantem A so lange durchgeführt wird, bis die Stimulation von der Steuer- und Analyseeinheit 10 als nicht erfolgreich klassifiziert wird, d. h., bis ein wie im Zusammenhang mit der Regelung nach Fig. 3 beschriebenes Abbruchkriterium erfüllt ist. Die Stimulation wird dann beendet. Die Vorrichtung 2 kann dann eine entsprechende Meldung ("Stimulation abgebrochen") an den Patienten, z. B. auf einem Display oder durch ein blinkendes Kontrolllämpchen oder dergleichen, erbringen. Diese Meldung kann auch per Funk, z. B. als SMS, E-Mail oder dergleichen, an den Arzt gehen. Als Alternative hierzu kann bei Erreichen des Abbruchkriteriums die Behandlung auch weiter durchgeführt werden, die entsprechende Meldung an den Patienten lautet dann z. B. "Bitte Arzt aufsuchen", und die SMS/E-Mail geht an den behandelnden Arzt, um ihn über die insuffiziente Therapie zu informieren.

Die nicht-invasive Stimulation, z. B. nicht-invasive CR-Stimulation, kann eine "open loop"-Stimulation oder eine "closed loop"-Stimulation sein. Im Fall der "closed loop"-Stimulation werden nicht implantierte Sensoren der Messeinheit 12, z. B. chronisch oder intermittent genutzte EEG- oder EMG-Elektroden oder MEG-Sensoren oder Akzelerometer (zur Detektion charakteristischer Bewegungsmuster wie Tremor, Akinese, epileptische Anfälle) oder Elektroden zur Messung des Hautleitwiderstands, und/oder weniger bevorzugt implantierte Sensoren, z. B. Tiefenelektroden, epikortikale Elektroden, verwandt, um die Stimulation zu steuern. Diese Sensoren können auch verwandt werden, (i) um die Regelung der Stimulationsphasen und Stimulationspausen, d. h. deren Anpassung an den therapeutischen Effekt, zu realisieren bzw. (ii) um Optimierungs- bzw. Abbruchkriterien wie oben in Zusammenhang mit den Fig. 3 und 4 beschrieben zu ermitteln. Es können aber auch für die Regelung der Längen Lstim und L_{Pause} der Stimulationsphasen und -pausen andere Sensoren bzw. andere Signale bzw. andere Signalbestandteile bzw. andere (ggfs. aus denselben Ausgangssignalen ermittelte) dynamische Biomarker für die "closed loop"-Stimulation verwandt werden. Bei der "closed loop"-Stimulation können z. B. relevante Stimulationsparameter, wie z. B. die CR-Stimulationsfrequenz, die Intensität der Einzelreize, die Dauer der Einzelreize, die Subgruppe von aktiven Aktoren einer größeren Gesamtgruppe, aber auch die Art der Stimulation, z. B. CR-Stimulation versus Stimulation mit Rausch-Signalen unterschiedlicher Art (Gausssches Rauschen, farbiges Rauschen etc.) sowie eine bedarfsgesteuerte Stimulation, bei der nur bei Vorliegen von gemessenen pathologischen Markern stimuliert wird, gesteuert werden.

Die einzelnen Komponenten der Vorrichtungen 1 und 2, insbesondere die Steuer- und Analyseeinheit 10, die Stimulationseinheit 11 und/oder die Messeinheit 12, können baulich voneinander getrennt sein. Die Vorrichtungen 1 und 2 können daher auch als Systeme aufgefasst werden.

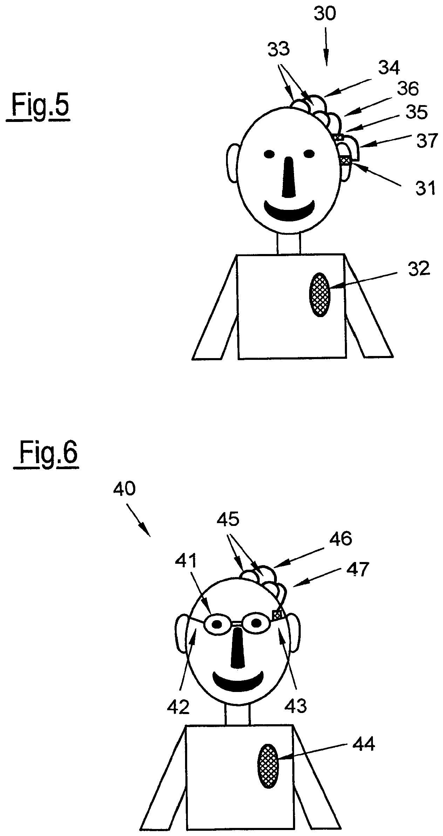

Fig. 5 zeigt schematisch eine Vorrichtung 30 zur nicht-invasiven akustischen Stimulation von Neuronen mit einer krankhaft synchronen und oszillatorischen neuronalen Aktivität gemäß einer Ausführungsform der Erfindung. Akustische Reize, insbesondere akustische CR-Reize, werden dem Patienten über Ohr- oder Kopfhörer 31 verabreicht, wobei ein Ohrhörer ein im Ohrkanal platzierter Lautsprecher ist. Die hierzu verwendeten Steuersignale werden von einer Steuer- und Analyseeinheit 32 generiert.

Nicht-invasiv fixierte EEG-Elektroden 33, die über ein Kabel 34 verbunden sind, dienen der "closed loop"-Stimulation und/oder der automatischen oben geschilderten Anpassung der Dauer von Stimulationspausen und Stimulationsphasen. Die entsprechende Verrechnung wird in einem kleinen Bauteil 35, das vorzugsweise einen Messverstärker enthält und über Kabel 36, 37 mit den EEG-Elektroden 33 bzw. dem Ohr- oder Kopfhörer 31 verbunden ist, und/oder in der eigentlichen, die Batterie bzw. den Akku beherbergenden Steuer- und Analyseeinheit 32 durchgeführt. Die Steuer- und Analyseeinheit 32 und das Bauteil 35 sind in der in Fig. 5 dargestellten Ausführungsform telemetrisch miteinander verbunden; in diesem Fall enthält Bauteil 35 (oder ein mit ihm über Kabel verbundenes Bauteil) ebenso eine Batterie bzw. einen Akku. Alternativ können die Steuer- und Analyseeinheit 32 und das Bauteil 35 auch über Kabel miteinander verbunden sein, so dass das Bauteil 35 über die Stromversorgung von der Steuer- und Analyseeinheit 32 gespeist wird.

Fig. 6 zeigt schematisch eine Vorrichtung 40 zur nicht-invasiven visuellen Stimulation von Neuronen mit einer krankhaft synchronen und oszillatorischen neuronalen Aktivität gemäß einer Ausführungsform der Erfindung. Bei dieser Ausgestaltung trägt der Patient eine Stimulationsbrille 41, die z. B. über Bügel 42 am Kopf des Patienten befestigt ist. Ein Bauteil 43 enthält eine Verrechnungs- und Telemetrieeinheit. Letztere dient der Verbindung mit der eigentlichen, die Batterie bzw. den Akku beherbergenden Steuer- und Analyseeinheit 44. Das Bauteil 43 und die Steuer- und Analyseeinheit 44 sind telemetrisch miteinander verbunden; in diesem Fall enthält Bauteil 43 (oder ein mit ihm über Kabel verbundenes Bauteil) ebenso eine Batterie bzw. einen Akku. Alternativ können das Bauteil 43 und die Steuer- und Analyseeinheit 44 auch über Kabel miteinander verbunden sein. Nicht-invasiv fixierte EEG-Elektroden 45 dienen der "closed loop"-Stimulation und/oder der oben beschriebenen, automatischen Anpassung der Dauer von Stimulationspausen und Stimulationsphasen. Die EEG-Elektroden 45 sind über Kabel 46, 47 mit dem Bauteil 43 verbunden.

Den von der Stimulationsbrille 41 erzeugten visuellen Reizen kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die visuellen Reize können je nach Ausgestaltung als Leuchtstärkenmodulation natürlicher visueller Reize, z. B. mittels einer homogenen oder segmentierten Transmissionsbrille, bei der spannungsabhängig die Transmission geregelt werden kann, als zusätzlich zu einem natürlichen visuellen Reiz auftretender, modulierter visueller Reiz, z. B. mittels einer partiell durchsichtigen Lichtbrille, oder als künstlicher visueller Helligkeitsreiz, z. B. mittels einer undurchsichtigen Lichtbrille, verabreicht werden. Die Stimulationsbrille 41 ist vorzugsweise in unterschiedliche Segmente unterteilt, deren Leuchtstärke bzw. Transmission bzw. Helligkeit getrennt gesteuert werden kann, um verschiedene Stellen der Retina unabhängig voneinander stimulieren zu können.

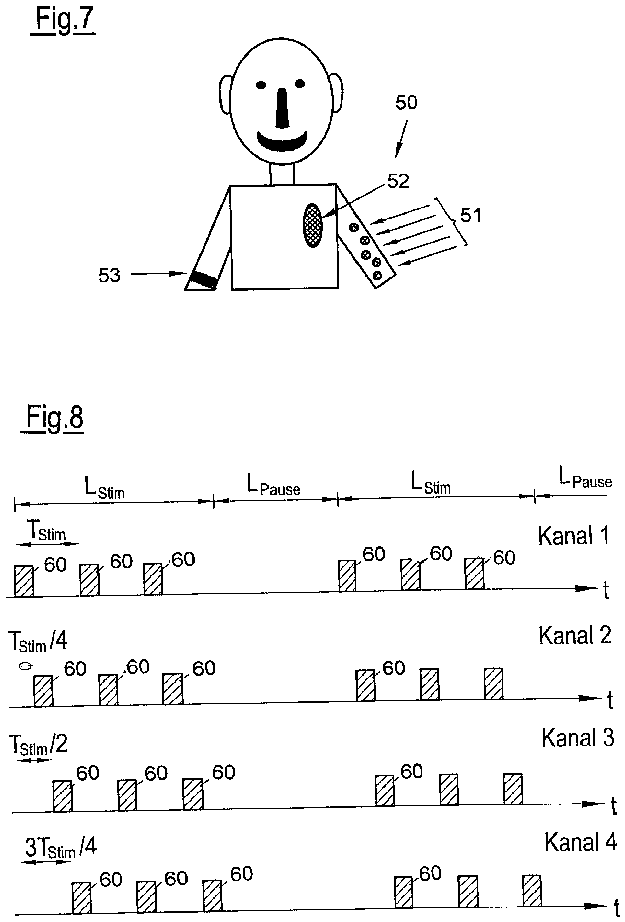

Fig. 7 zeigt schematisch eine Vorrichtung 50 zur nicht-invasiven taktilen, vibratorischen, thermischen, elektrischen transkutanen und/oder magnetischen Stimulation und/oder Ultraschall-Stimulation von Neuronen mit einer krankhaft synchronen und oszillatorischen neuronalen Aktivität gemäß einer Ausführungsform der Erfindung. Die Vorrichtung 50 umfasst eine Stimulationseinheit 51, eine die Stimulationseinheit 51 ansteuernde Steuer- und Analyseeinheit 52 und ein Akzelerometer (Beschleunigungsmesser) 53 zum Aufnehmen von Messsignalen. Die Stimulationseinheit 51 und das Akzelerometer 53 können mit der Steuer- und Analyseeinheit 52 telemetrisch oder über Kabel verbunden sein.

Die Stimulationseinheit 51 umfasst eine Mehrzahl von Stimulationselementen zur Erzeugung von taktilen, vibratorischen, thermischen, elektrischen transkutanen und/oder magnetischen Reizen und/oder Ultraschall-Reizen. Die Stimulationselemente sind derart ausgestaltet, dass sie auf die Haut des Patienten aufgesetzt werden können. Je nach Erkrankung bzw. betroffenen Körperpartien werden die Stimulationselemente in einer geeigneten Anordnung auf der Haut des Patienten befestigt, beispielsweise am Arm, am Bein, an der Hand und/oder am Fuß des Patienten. Die Mehrzahl von Stimulationselementen ermöglicht es, unterschiedliche rezeptive Bereiche der Haut über die einzelnen Stimulationselemente zeitlich und räumlich koordiniert zu stimulieren.

Bei der Applikation akustischer oder visueller Reize werden diese über mindestens ein Ohr bzw. mindestens ein Auge des Patienten aufgenommen. Die taktilen, vibratorischen, thermischen, elektrischen transkutanen und/oder magnetischen Reize und/oder Ultraschall-Reize werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize wirken. Die vibratorischen Reize zielen vorwiegend auf die Tiefensensibilität ab. Die vibratorischen Reize können von in der Haut, den Muskeln, dem Subkutangewebe und/oder den Sehnen des Patienten gelegenen Rezeptoren aufgenommen werden. Als Rezeptoren für die vibratorischen Reize seien beispielhaft die Vater-Pacini-Körperchen genannt, die Vibrationsempfindungen und Beschleunigungen vermitteln. Die thermischen Reize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer. Die elektrischen transkutanen und magnetischen Reize sowie die Ultraschall-Reize wirken nicht spezifisch auf nur eine Gruppe von in oder unter der Haut gelegenen Rezeptoren. Mittels der elektrischen transkutanen Reize kann das Zielgebiet daher über unterschiedliche Kanäle stimuliert werden.

Die gezielte Stimulation bestimmter Bereiche des Gehirns oder Rückenmarks wird durch die tonotope bzw. somatotope Zuordnung von Körperregionen zu diesen Bereichen ermöglicht. Beispielsweise werden akustische Reize im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Bei der visuellen Stimulation werden unterschiedliche Stellen im Gesichtsfeld über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Folglich können mit den an unterschiedlichen räumlichen Orten applizierten Reizen jeweils unterschiedliche Neuronen stimuliert werden.

Aufgrund der somatotopen Gliederung der Nervenleitungsbahnen und zugehörigen Hirngebiete werden des Weiteren durch taktile, vibratorische, thermische, elektrische transkutane und/oder magnetische Reize und/oder Ultraschall-Reize, die an unterschiedlichen Stellen der Haut appliziert werden, unterschiedliche Neuronen stimuliert. Bei diesen Stimulationsformen können die Stimulationselemente beispielsweise am Fuß, Unterschenkel und Oberschenkel oder aber an der Hand, dem Unterarm und Oberarm des Patienten angebracht werden, um dadurch bestimmte Neuronen stimulieren zu können.

Die vorstehend beschriebenen Stimulationseinheiten können demnach unterschiedliche Bereiche des Gehirns oder Rückenmarks separat stimulieren, indem die applizierten Reize über Nervenleitungen an unterschiedliche Zielgebiete, die im Gehirn und/oder Rückenmark liegen, weitergeleitet werden. Die Zielgebiete können während der Stimulation mit eventuell unterschiedlichen und/oder zeitversetzten Reizen stimuliert werden.

Die hierin beschriebenen Vorrichtungen, insbesondere die Vorrichtungen 1, 2, 30, 40 und 50, können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, chronische Schmerzsyndrome, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden.

Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 50 Hz, kann aber auch außerhalb dieses Bereichs liegen. Bei gesunden Menschen feuern die Neuronen hingegen qualitativ anders, z. B. auf unkorrelierte Weise.

Bei der oben erwähnten CR-Stimulation werden die dem Patienten verabreichten Reize über das Nervensystem an eine Neuronenpopulation im Hirn und/oder Rückenmark weitergeleitet, die eine krankhaft synchrone und oszillatorische neuronale Aktivität aufweist. Die Reize sind so ausgestaltet, dass die krankhaft synchrone Aktivität der Neuronenpopulation desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize bewirken in der Neuronenpopulation ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation mittels einer gezielten Stimulation kontrolliert. Ferner wird die krankhafte Neuronenpopulation mittels mehrerer Stimulationskontakte der Stimulationseinheit an unterschiedlichen Stellen stimuliert, so dass die Phase der neuronalen Aktivität der krankhaften Neuronenpopulation an den unterschiedlichen Stimulationsstellen zu unterschiedlichen Zeitpunkten zurückgesetzt werden kann. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb jeder der Subpopulationen sind die Neuronen nach dem Zurücksetzen der Phase weiterhin synchron und feuern auch weiterhin mit derselben pathologischen Frequenz, aber jede der Subpopulationen weist bezüglich ihrer neuronalen Aktivität die Phase auf, die ihr durch den von dem jeweiligen Stimulationskontakt generierten Reiz aufgezwungen wurde. Dies bedeutet, dass die neuronalen Aktivitäten der einzelnen Subpopulationen nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize nicht sofort vorhanden, sondern stellt sich meist innerhalb weniger Perioden oder gar in weniger als einer Periode der pathologischen Frequenz ein.

Fig. 8 zeigt ein Beispiel einer CR-Stimulation mit insgesamt vier Kanälen. Über jeden der vier Kanäle wird eine Subpopulation der krankhaften Neuronenpopulation stimuliert. In jedem der vier Kanäle werden pulsförmige Reize 60 in einer Sequenz periodisch mit der Periode Tstim appliziert, wobei die Periode T_{Stim} nahe bei der mittleren Periode der pathologischen Oszillation der Neuronenpopulation liegt bzw. um bis zu ± 5%, ± 10% oder ± 20% von dem Literaturwert abweicht (typischerweise liegt fstim = 1 /Tstim im Bereich von 1 bis 30 Hz). Die Reize 60 bewirken eine Phasenrücksetzung der neuronalen Aktivität der jeweils stimulierten Subpopulation. Ferner beträgt die zeitliche Verzögerung zwischen den Sequenzen benachbarter Kanäle T_{Stim}/4, da vier Kanäle vorhanden sind. Für den allgemeinen Fall von N Kanälen würde die zeitliche Verzögerung benachbarter Kanäle T_{Stim}/N betragen (von diesem Wert kann auch um z. B. bis zu ± 5%, ± 10% oder ± 20% abgewichen werden). Ferner muss die Reihenfolge der Reizverabreichung über die N Kanäle nicht in jedem Stimulationszyklus identisch sein, sondern kann z. B. auch von Stimulationszyklus zu Stimulationszyklus randomisiert variiert werden.

Je nach Modalität der Reize 60 sind diese so ausgestaltet, dass die jeweils gewünschte Subpopulation stimuliert wird. Im Fall der akustischen Stimulation weisen die Reize 60 in einem jeweiligen Kanal beispielsweise eine bestimmte Frequenz oder ein bestimmtes Frequenzgemisch auf, das so ausgewählt ist, dass aufgrund der tonotopen Organisation des auditorischen Cortex eine bestimmte Subpopulation stimuliert wird. Im Fall der visuellen Stimulation entsprechen die Kanäle unterschiedlichen Stellen im Gesichtsfeld, welche über die Linse des Auges auf unterschiedliche Stellen der Retina abgebildet werden, welche wiederum über den Sehnerv mit unterschiedlichen Neuronen im Gehirn verbunden sind. Im Fall von taktilen, vibratorischen, thermischen, elektrischen transkutanen und/oder magnetischen Reizen 60 und/oder Ultraschall-Reizen 60 stehen die Kanäle für unterschiedliche Stellen der Haut, an denen die Reize 60 appliziert werden und die mit den gewünschten Subpopulationen im Gehirn oder Rückenmark über das Nervensystem verbunden sind.

Ferner sind in Fig. 8 die Längen Lstim der Stimulationsphasen und die Längen L_{Pause} der Stimulationspausen dargestellt, die wie oben beschrieben eingestellt oder geregelt werden können. Zu beachten ist, dass in Fig. 8 die Längen L_{Stim} und L_{Pause} und die Längen der phasenrücksetzenden Reize 60 nicht maßstabsgetreu wiedergegeben sind.

Es sei darauf hingewiesen, dass auch bei herkömmlichen Stimulationsverfahren Pausen eingehalten werden können, in denen keine Reize appliziert werden. Beispielsweise kann bei der CR-Stimulation für n Zyklen stimuliert und für die folgenden m Zyklen nicht stimuliert werden und dieses Stimulationsmuster periodisch fortgeführt werden, wobei n und m kleine ganze Zahlen sind. Derartige Pausen können erfindungsgemäß auch während der Stimulationsphasen der Länge L_{Stim} eingehalten werden. Die erfindungsgemäßen Stimulationspausen der Länge L_{Pause} unterscheiden sich jedoch von den Pausen während der Stimulationsphasen darin, dass sie nur dann eingehalten werden, wenn zuvor festgestellt wurde, dass der durch die Stimulation erzielte Stimulationserfolg nicht ausreichend ist, und/oder Nebenwirkungen auftreten und/oder die Stimulationseinheit im Körper des Patienten ungünstig platziert ist.

Anstelle der CR-Stimulation können auch anderen Stimulationsformen verwendet werden, sofern sich mit diesen Stimulationsformen bei der Desynchronisation von krankhaft aktiven Neuronenpopulationen lang anhaltende therapeutische Effekte erzielen lassen.

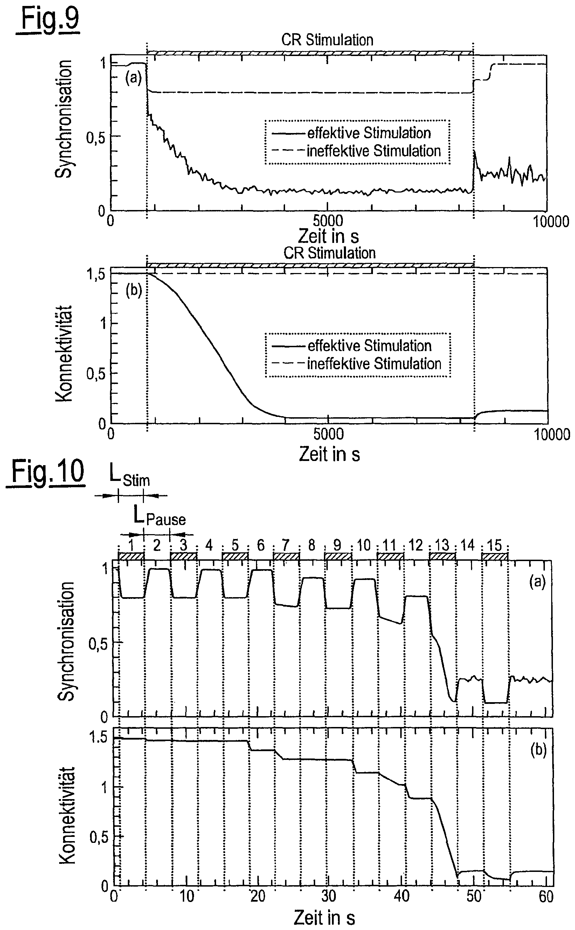

In den Fig. 9 bis 15 werden die mit der hierin beschriebenen Erfindung erzielbaren Effekte anhand von Simulationsergebnissen veranschaulicht.

In den Fig. 9(a) und 9(b) sind der Grad der Synchronisation und die synaptische Konnektivität einer Neuronenpopulation mit einer krankhaft synchronen und oszillatorischen neuronalen Aktivität vor, während und nach einer CR-Stimulation dargestellt. Die in den beiden Darstellungen oben eingezeichneten horizontalen Balken geben den Zeitraum an, in dem die CR-Stimulation angewendet wird.

Wie die Fig. 9(a) und 9(b) zeigen, bewirkt eine effektive CR-Stimulation eine rasche Desynchronisation der Neuronenpopulation sowie eine starke Verringerung der Konnektivität. Jedoch kann sich unter bestimmten Umständen ein nur geringer Stimulationserfolg einstellen, was sich daran ablesen lässt, dass sich der Synchronisationsgrad und die Konnektivität innerhalb der stimulierten Neuronenpopulation trotz der CR-Stimulation nur geringfügig verringern.

Mit den oben beschriebenen Vorrichtungen 1 und 2 lässt sich die Effizienz der CR-Stimulation durch die Einfügung von Stimulationspausen bei geringen Reizstärken verbessern. Weiterhin können z. B. im Fall von Nebenwirkungen Stimulationspausen eingefügt werden, um eine effiziente Stimulation bei geringen Reizstärken zu ermöglichen.

Nebenwirkungen hängen von der jeweiligen Erkrankung und dem jeweils gewählten Zielgebiet ab. Es können z. B. Dyskinesien auftreten, die sich durch eine Koaktivierung (anstatt einer alternierenden Aktivierung) von antagonistischen Muskeln (z. B. Beugern und Streckern) zeigen. Nebenwirkungen können sich auch zeigen durch eine stimulationsabhängige Zunahme von synchroner Aktivität in entsprechenden Sensoren.

Fig. 10(a) und 10(b) zeigen die Ergebnisse einer Stimulation, die alternierende Stimulationsphasen, in denen eine CR-Stimulation durchgeführt wird, und Stimulationspausen, in denen keine Stimulation durchgeführt wird, umfasst. Die Stimulationsphasen sind in den Fig. 10(a) und 10(b) durch horizontale Balken gekennzeichnet. Die Längen L_{Stim} und L_{Pause} der Stimulationsphasen und -pausen sind in dem vorliegenden Beispiel gleich lang und betragen jeweils 3.600 s. Bis auf die Stimulationspausen wurden für die in den Fig. 10(a) und 10(b) dargestellten Simulationen die gleichen Stimulationsparameter verwendet wie für die in den Fig. 9(a) und 9(b) gezeigte Simulation der ineffektiven Stimulation. Das Einschieben der Stimulationspausen führt zu einer deutlichen Abnahme des Synchronisationsgrads und der Konnektivität. Das Einschieben der Stimulationspausen bewirkt folglich, dass eine ansonsten wirkungslose Stimulation effektiv ist. Ferner lassen sich mit dieser Stimulationsform lang anhaltende therapeutische Effekte erzielen. Auch nach dem kompletten Ausschalten der Stimulation verbleiben der Synchronisationsgrad und die Konnektivität auf einem sehr geringen Niveau.

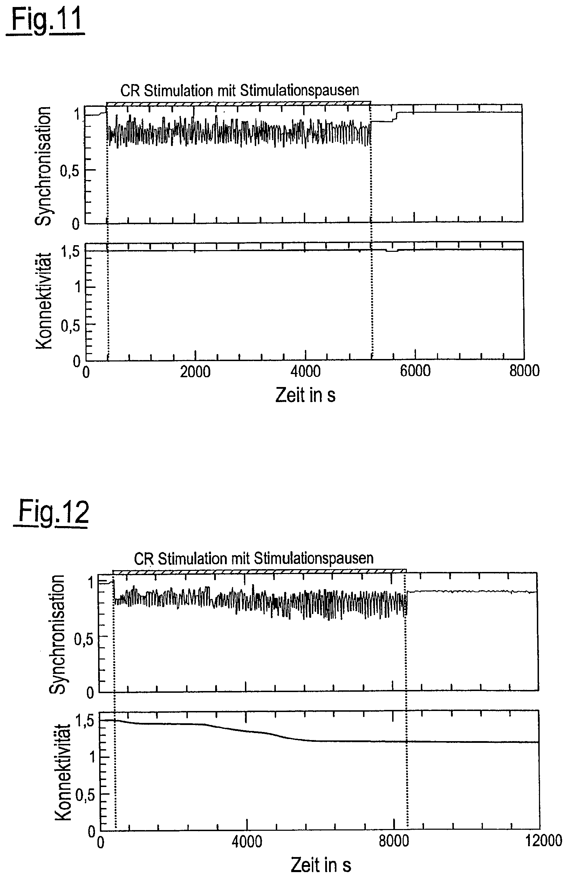

Für den Erfolg der erfindungsgemäßen Stimulationsform ist es wichtig, geeignete Längen Lstim und L_{Pause} für die Stimulationsphasen und -pausen zu ermitteln. Die Fig. 11 bis 14 zeigen die Ergebnisse verschiedener Simulationen, für die bei ansonsten gleichen Stimulationsparametern unterschiedliche Werte für L_{Stim} und L_{Pause} verwendet wurden. Diese Werte sind in der nachfolgenden Tabelle aufgeführt.

| | L_{Stim} | L_{Pause} |
|---|---|---|
| Fig. 11 | 24 s | 24 s |
| Fig. 12 | 24 s | 56 s |
| Fig. 13 | 24 s | 80 s |
| Fig. 14 | 960 s | 720 s |

In den Fig. 11 bis 14 sind durch die horizontalen Balken die Zeiträume gekennzeichnet, in denen die erfindungsgemäße CR-Stimulation mit einander abwechselnden Stimulationsphasen und -pausen durchgeführt wird.

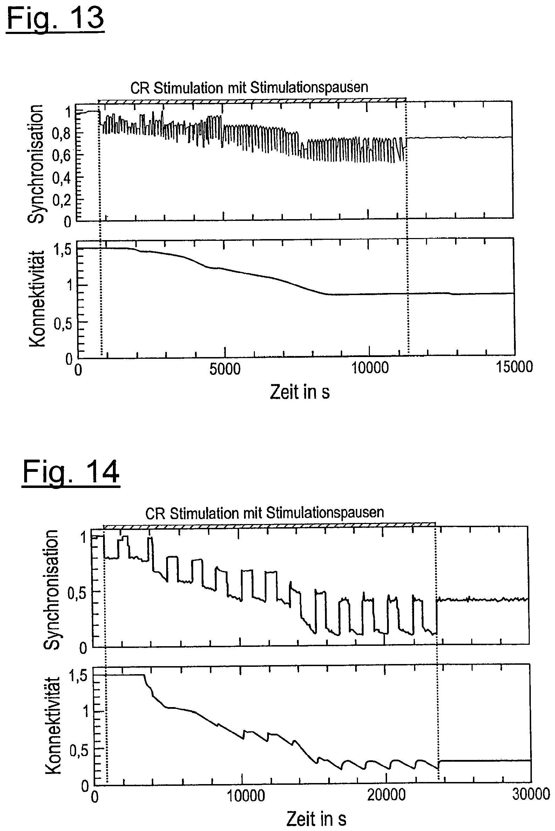

Für nur sehr kurze Stimulationsphasen und -pausen stellt sich ein nur sehr geringer Effekt ein (vgl. Fig. 11), der darüber hinaus nach dem Beenden des Stimulationsvorgangs von nur kurzer Dauer ist. Bessere Ergebnisse werden erzielt, wenn bei gleichbleibender Länge Lstim der Stimulationsphasen die Länge L_{Pause} der Stimulationspausen vergrößert wird (vgl. Fig. 12 und 13). Die besten Ergebnisse werden bei den hier dargestellten Simulationen für relativ große Werte von mehreren Minuten für die Längen Lstim und L_{Pause} erzielt (vgl. Fig. 14).

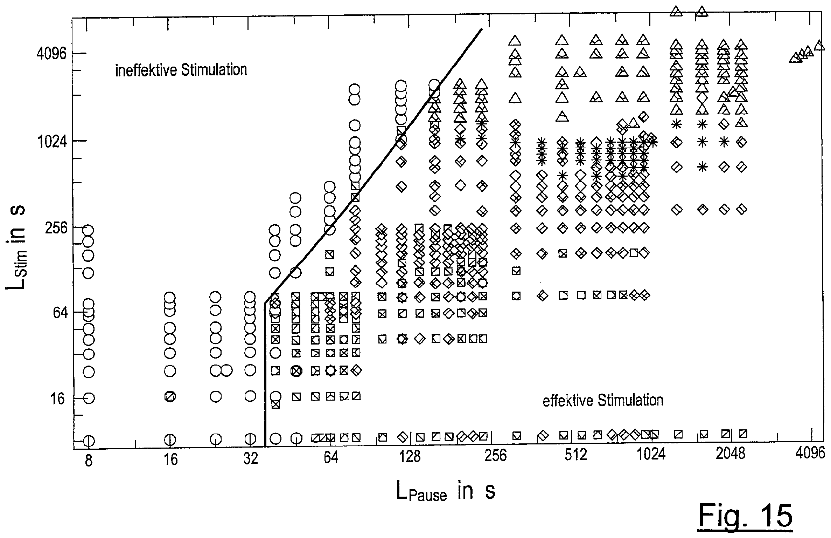

In Fig. 15 sind die Ergebnisse von unterschiedlichen erfindungsgemäßen CR-Stimulationen in einer (L_{Stim}, L_{Pause})-Ebene dargestellt. Die Kreis-Symbole zeigen ineffektive Stimulationen an, alle anderen Symbole stehen für effektive Stimulationen. Ferner ist die (L_{Stim}, L_{Pause})-Ebene in Fig. 14 durch eine Linie in einen Bereich ineffektiver Stimulation und einen Bereich effektiver Stimulation unterteilt. Wie sich Fig. 14 entnehmen lässt, sind Stimulationen mit nur kurzen Werten für Lstim und L_{Pause} sowie Stimulationen, bei denen L_{Stim} im Vergleich zu L_{Pause} zu lang ist, ineffektiv.

Die besten Stimulationsergebnisse wurden erzielt für Parameterwerte aus dem oberen rechten Bereich der (L_{Stim}, L_{Pause})-Ebene.

## Patentansprüche

1. Vorrichtung (2) zur Unterdrückung einer krankhaft synchronen und oszillatorischen neuronalen Aktivität, umfassend
- eine nicht-invasive Stimulationseinheit (11) zur Stimulation von Neuronen im Hirn und/oder Rückenmark des Patienten, die eine krankhaft synchrone und oszillatorische neuronale Aktivität aufweisen, mit Reizen (22), wobei die Reize (22) dazu ausgelegt sind, bei einer Verabreichung an den Patienten die krankhaft synchrone und oszillatorische neuronale Aktivität zu unterdrücken,
- eine Messeinheit (12) zum Aufnehmen von Messsignalen (23), die eine neuronale Aktivität der stimulierten Neuronen wiedergeben, und
- eine Steuer- und Analyseeinheit (10) zur Steuerung der Stimulationseinheit (11) und zur Analyse der Messsignale (23), wobei die Steuer- und Analyseeinheit (10) derart ausgestaltet ist, dass sie
- die Stimulationseinheit (11) derart ansteuert, dass die Stimulationseinheit (11) Reize (22) appliziert,
- anhand der in Reaktion auf die Applikation der Reize (22) aufgenommenen Messsignale (23) den Stimulationserfolg überprüft,
**dadurch gekennzeichnet, dass** die Steuer- und Analyseeinheit (10) derart ausgestaltet ist, dass sie
- falls der Stimulationserfolg nicht ausreichend ist, eine oder mehrere Stimulationspausen in die Applikation der Reize (22) einfügt oder eine oder mehrere Stimulationspausen verlängert, wobei während der Stimulationspausen keine Reize appliziert werden, welche die krankhaft synchrone und oszillatorische neuronale Aktivität unterdrücken könnten.

2. Vorrichtung (2) nach Anspruch 1, wobei die Reize (22) akustische, visuelle, taktile, vibratorische, thermische, olfaktorische, gustatorische, magnetische transkranielle, elektrische transkranielle, magnetische transkutane und/oder elektrische transkutane Reize und/oder Ultraschall-Reize sind.

3. Vorrichtung (2) nach Anspruch 1, wobei die Steuer- und Analyseeinheit (10) ferner derart ausgestaltet ist, dass sie die Dauer der Stimulationspausen insbesondere schrittweise verlängert, bis die Steuer- und Analyseeinheit (10) feststellt, dass der Stimulationserfolg ausreichend ist.

4. Vorrichtung (2) nach Anspruch 3, wobei die Steuer- und Analyseeinheit (10) ferner derart ausgestaltet ist, dass sie neben der Dauer der Stimulationspausen die Dauer der Stimulationsphasen insbesondere schrittweise verlängert, bis die Steuer- und Analyseeinheit (10) feststellt, dass der Stimulationserfolg ausreichend ist.

5. Vorrichtung (2) nach einem der vorhergehenden Ansprüche, wobei
- die Stimulationsphasen jeweils eine Dauer Lstim und die zwischen aufeinander folgenden Stimulationsphasen eingehaltenen Stimulationspausen jeweils eine Dauer L_{Pause} aufweisen und A = Lstim = L_{Pause} oder A = L_{Stim}/L_{Pause} gilt, und
- die Steuer- und Analyseeinheit (10) ferner derart ausgestaltet ist, dass sie, falls der Stimulationserfolg nicht ausreichend ist, A insbesondere schrittweise vergrößert.

6. Vorrichtung (2) nach Anspruch 5, wobei die Steuer- und Analyseeinheit (10) ferner derart ausgestaltet ist, dass sie, falls der Stimulationserfolg nicht ausreichend ist, A für A = L_{Stim}/L_{Pause} von 1/n auf n insbesondere schrittweise vergrößert, wobei n insbesondere eine Zahl im Bereich von 2 bis 10 ist.

7. Vorrichtung (2) nach Anspruch 5 oder 6, wobei die Steuer- und Analyseeinheit (10) ferner derart ausgestaltet ist, dass sie A so lange konstant hält, bis die Steuer- und Analyseeinheit (10) feststellt, dass der Stimulationserfolg nicht ausreichend ist.

8. Vorrichtung (2) nach einem der Ansprüche 5 bis 7, wobei die Steuer- und Analyseeinheit (10) ferner derart ausgestaltet ist, dass sie A bei nicht ausreichendem Stimulationserfolg so lange vergrößert, bis die Steuer- und Analyseeinheit (10) feststellt, dass der Stimulationserfolg ausreichend ist oder ein Abbruchkriterium erfüllt ist.

9. Vorrichtung (2) nach einem der vorhergehenden Ansprüche, wobei die Stimulationseinheit (11) derart ausgestaltet ist, dass sie während der Stimulationsphasen eine "Coordinated Reset"-Stimulation durchführt, wobei die Phasen der neuronalen Aktivität von mehreren Subpopulationen einer stimulierten Neuronenpopulation mit einer krankhaft synchronen und oszillatorischen neuronalen Aktivität zu unterschiedlichen Zeitpunkten zurückgesetzt werden.

10. Vorrichtung (2) nach einem der vorhergehenden Ansprüche, wobei die Dauer einer Stimulationspause mindestens 3 Minuten beträgt.

11. Vorrichtung (2) nach einem der vorhergehenden Ansprüche, wobei die Dauer einer Stimulationspause der Dauer von mindestens 200 oder mindestens 1.000 Perioden der krankhaft synchronen und oszillatorischen neuronalen Aktivität entspricht.

## Claims

1. An apparatus (2) for suppressing a pathologically synchronous and oscillatory neuronal activity comprising
- a non-invasive stimulation unit (11) for stimulating neurons in the brain and/or spinal cord of the patient that exhibit a pathologically synchronous and oscillatory neuronal activity, using stimuli (22), wherein the stimuli (22) are adapted to suppress the pathologically synchronous and oscillatory neuronal activity via an administration to the patient;
- a measuring unit (12) for recording measured signals (23) which reproduce a neuronal activity of the stimulated neurons; and
- a control and analysis unit (10) for controlling the stimulation unit (11) and for analyzing the measured signals (23), wherein the control and analysis unit (10) is configured such that it
- controls the stimulation unit (11) such that the stimulation unit (11) applies stimuli (22);
- checks the stimulation success with reference to the measured signals (23) recorded in response to the application of the stimuli (22); and,
- if the stimulation success is not sufficient, inserts one or more stimulation breaks into the application of the stimuli (22) or extends one or more stimulation breaks, wherein no stimuli are applied during the stimulation breaks that can suppress the pathologically synchronous and oscillatory neuronal activity.

2. An apparatus (2) in accordance with claim 1, wherein the stimuli (22) are acoustic, visual, tactile, vibratory, thermal, olfactory, gustatory, magnetic transcranial, electrical transcranial, magnetic transcutaneous and/or electrical transcutaneous stimuli and/or ultrasound stimuli.

3. An apparatus (2) in accordance with claim 1, wherein the control and analysis unit (10) is furthermore configured such that it extends the duration of the stimulation breaks, in particular incrementally, until the control and analysis unit (10) determines that the stimulation success is sufficient.

4. An apparatus (2) in accordance with claim 3, wherein the control and analysis unit (10) is furthermore configured such that it extends the duration of the stimulation phases, in addition to the duration of the stimulation breaks, in particular incrementally, until the control and analysis unit (10) determines that the stimulation success is sufficient.

5. An apparatus (2) in accordance with any one of the preceding claims, wherein
- the stimulation phases each have a duration L_{Stim} and the stimulation breaks observed between successive stimulation phases each have a duration L_{Break} and A = L_{Stim} = L_{Break} or A = L_{Stim}/L_{Break} applies; and
- the control and analysis unit (10) is furthermore configured such that it increases A, in particular incrementally, if the stimulation success is not sufficient.

6. An apparatus (2) in accordance with claim (5), wherein the control and analysis unit (10) is furthermore configured such that it increases A for A = L_{Stim}/L_{Break} from 1/n to n, in particular incrementally, if the stimulation success is not sufficient, where n is in particular a number in the range from 2 to 10.

7. An apparatus (2) in accordance with claim 5 or claim 6, wherein the control and analysis unit (10) is furthermore configured such that it keeps A constant for so long until the control and analysis unit (10) determines that the stimulation success is not sufficient.

8. An apparatus (2) in accordance with any one of the claims 5 to 7, wherein the control and analysis unit (10) is furthermore configured such that it increases A if the stimulation success is insufficient for so long until the control and analysis unit (10) determines that the stimulation success is sufficient or until an abort criterion is satisfied.

9. An apparatus (2) in accordance with any one of the preceding claims, wherein the stimulation unit (11) is configured such that it carries out a "coordinated reset" stimulation during the stimulation phases, wherein the phases of neuronal activity of a plurality of subpopulations of a stimulated neural population having a pathologically synchronous and oscillatory neuronal activity are reset at different points in time.

10. An apparatus (2) in accordance with any one of the preceding claims, wherein the duration of a stimulation break amounts to at least 3 minutes.

11. An apparatus (2) in accordance with any one of the preceding claims, wherein the duration of a stimulation break corresponds to the duration of at least 200 or at least 1,000 periods of the pathologically synchronous and oscillatory neuronal activity.

## Revendications

1. Dispositif (2) pour la suppression d'une activité neuronale pathologiquement synchrone et oscillatoire, comprenant
- un module de stimulation non invasif (11) pour la stimulation de neurones dans le cerveau et/ou la moëlle épinière du patient qui présentent une activité neuronale pathologiquement synchrone et oscillatoire, avec des stimuli (22), dans lequel les stimuli (22) sont conçus pour supprimer lors d'une administration aux patients l'activité neuronale pathologiquement synchrone et oscillatoire,
- un module de mesure (12) pour l'enregistrement de signaux de mesure (23) qui restituent une activité neuronale des neurones stimulés, et
- un module de commande et d'analyse (10) pour la commande du module de stimulation (11) et pour l'analyse des signaux de mesure (23), dans lequel le module de commande et d'analyse (10) est configuré de telle sorte qu'il
- commande le module de stimulation (11) de telle sorte que le module de stimulation (11) applique des stimuli (22),
- vérifie le succès de stimulation à l'aide des signaux de mesure (23) enregistrés en réaction à l'application des stimuli (22),
**caractérisé en ce que** le module de commande et d'analyse (10) est configuré de telle sorte qu'il
- introduit une ou plusieurs pauses de stimulation dans l'application des stimuli (22) ou prolonge une ou plusieurs pauses de stimulation au cas où le succès de stimulation n'est pas suffisant, dans lequel aucun stimulus qui pourrait supprimer l'activité neuronale pathologiquement synchrone et oscillatoire n'est appliqué pendant les pauses de stimulation.

2. Dispositif (2) selon la revendication 1, dans lequel les stimuli (22) sont des stimuli acoustiques, visuels, tactiles, vibratoires, thermiques, olfactifs, gustatifs, magnétiques transcrâniens, électriques transcrâniens, magnétiques transcutanés et/ou électriques transcutanés et/ou des stimuli ultrasoniques.

3. Dispositif (2) selon la revendication 1, dans lequel le module de commande et d'analyse (10) est en outre configuré de telle sorte qu'il prolonge notamment progressivement la durée des pauses de stimulation jusqu'à ce que le module de commande et d'analyse (10) constate que le succès de stimulation est suffisant.

4. Dispositif (2) selon la revendication 3, dans lequel le module de commande et d'analyse (10) est en outre configuré de telle sorte qu'il prolonge notamment progressivement outre la durée des pauses de stimulation la durée des phases de stimulation jusqu'à ce que le module de commande et d'analyse (10) constate que le succès de stimulation est suffisant.

5. Dispositif (2) selon une des revendications précédentes, dans lequel
- les phases de stimulation présentent chacune une durée L_{Stim} et les pauses de stimulation respectées entre des phases de stimulation successives chacune une durée L_{Pause}, et il est valable A = L_{Stim} = L_{Pause} ou A = L_{Stim}/L_{Pause}, et
- le module de commande et d'analyse (10) est en outre configuré de telle sorte qu'il agrandit notamment progressivement A au cas où le succès de stimulation n'est pas suffisant.

6. Dispositif (2) selon la revendication 5, dans lequel le module de commande et d'analyse (10) est en outre configuré de telle sorte qu'il agrandit notamment progressivement A pour A = L_{Stim}/L_{Pause} de 1/n à n au cas où le succès de stimulation n'est pas suffisant, dans lequel n est notamment un nombre dans la région de 2 à 10.

7. Dispositif (2) selon la revendication 5 ou 6, dans lequel le module de commande et d'analyse (10) est en outre configuré de telle sorte qu'il maintient A constant jusqu'à ce que le module de commande et d'analyse (10) constate que le succès de stimulation n'est pas suffisant.

8. Dispositif (2) selon une des revendications 5 à 7, dans lequel le module de commande et d'analyse (10) est en outre configuré de telle sorte qu'il agrandit A en cas de succès de stimulation non suffisant jusqu'à ce que le module de commande et d'analyse (10) constate que le succès de stimulation est suffisant ou un critère d'interruption est satisfait.

9. Dispositif (2) selon une des revendications précédentes, dans lequel le module de stimulation (11) est configuré de telle sorte qu'il réalise une stimulation de « réinitialisation coordonnée » pendant les phases de stimulation, dans lequel les phases de l'activité neuronale de plusieurs sous-populations d'une population de neurones stimulée avec une activité neuronale pathologiquement synchrone et oscillatoire sont réinitialisées à différents moments.

10. Dispositif (2) selon une des revendications précédentes, dans lequel la durée d'une pause de stimulation se monte à au moins 3 minutes.

11. Dispositif (2) selon une des revendications précédentes, dans lequel la durée d'une pause de stimulation correspond à la durée d'au moins 200 ou d'au moins 1000 périodes de l'activité neuronale pathologiquement synchrone et oscillatoire.
